# EUROPEAN PATENT SPECIFICATION

(11) **EP 1 794 180 B1**
(45) Date of publication and mention of the grant of the patent: **04.06.2008**
(21) Application number: 05786327.6
(22) Date of filing: 20.09.2005
(51) Int. Cl.: C07K 1/06, C07K 7/54, C07C 29/10, C07C 51/09, C07C 209/62

(54) **PEPTIDE CYCLISATION**
PEPTIDCYCLISIERUNG
CYCLISATION DE PEPTIDES

(30) Priority: 20.09.2004 EP 04022310; 11.07.2005 EP 05014954
(43) Date of publication of application: 13.06.2007
(73) Proprietor: Lonza AG, 4052 Basel (CH)
(72) Inventor: GIRAUD, Matthieu, CH-1950 Sion (CH); WERBITZKY, Oleg, CH-3968 Veyras (CH); WILLINER, Michaela, CH-3902 Glis (CH)
(74) Representative: Riegler, Norbert Hermann
(86) International application number: PCT/EP2005/010133
(87) International publication number: WO 2006/032457

(56) References cited:
- WO-A-01/00224
- RIJKERS DIRK T S ET AL: "An optimized solid phase synthesis strategy: Including on-resin lactamization: Of astressin, its retro-, inverso-, and retro-inverso isomers as corticotropin releasing factor antagonists" BIOPOLYMERS, vol. 63, no. 2, February 2002 (2002-02), pages 141-149, XP002358605 ISSN: 0006-3525 cited in the application
- PAUL,F.;PATT,J.;HARTWIG,J.F.: "structural charachterization and simple synthesis of {Pd[P(o-Tol)3]2},dimeric Palladium(II) complexes obtained by oxidative addition of aryl bromides, and corresponding monometallic amine complexes" ORGANOMETALLICS, vol. 14, 1995, pages 3030-3039, XP002358606 cited in the application

## Description

The present invention relates to a method of synthesis for a cyclic peptide, namely a cyclic peptide comprising ring closure of the carboxy group of the side chain of a one amino acid residue and the amino group of a side chain of a second amino acid residue.

On-resin cyclisation of a peptide by lactam formation in between the ω-carboxyl group of an amino acid side chain (i.e., the carboxyl group of a side chain irrespective of the carbon chain length), typically an aspartyl or glutamyl resiude, and the ω-amino group of an amino acid side chain (i.e., the amino group of a side chain irrespective of the carbon chain length), typcially a lysine residue, has been described.

Rijkers et al. (An optimized solid phase strategy - including on-resin lactamization- of Astressin, its retro-, inverso- and retro-inverso isomers, 2002, Biopolymers 63, 141-149) describe latamisation of Boc-protected 41-mer bound to Rink amide resin. Positions 30 (Glu) and 33 (Lys) are deprotected in a single step by Pd(0) catalyzed removal of allyl and alloc protection groups and are subsequently cyclised by the presence of BOP/HOBt and Hünig base in N-methylpyrrolidone. The peptide was synthesized using a standard orthogonal protection scheme employing Fmoc except for the last residue, which was Boc protected. Subsequently, the N-terminal Boc protection group was removed by acid TFA treatment, simultaneously liberating the peptide from the resin.

A disadvantage of this method is that termination of full-length peptide synthesis is a pre-requisite for subsequent cyclisation of a segment of the peptide. Hence the more valuable full-length peptide is subject to yield losses due to unwanted side-reactions (pyroglutamate formation) or incomplete allyl/alloc deprotection. Further, it is not always desirable to use an acid-labile protection group whilst on resin, since this prevents subsequent further derivatisation of the peptide on-resin, e.g. such as N-terminal blocking by acetylation. No reaction may be carried out prior to N-terminal acetylation since this renders the terminal Nα vulnerable to epimerisation at the chiral Cα.

Kates et al. (A novel, convenient three dimensional orthogonal strategy for solid-phase synthesis of cyclic peptides, 1993, Tetrahedron Letters 34:1549-1552) describes head-to-tail cyclisation of a decameric peptide by side chain anchoring of a C-terminal aspartyl or glutamyl residue to different resin handles which residue is protected at its Ca by an allyl ester protection group. After linear solid phase Fmoc synthesis of the complete peptide chain, the allyl ester moiety is removed by Pd-catalysis, followed by the sequence of Nα-Fmoc removal and subsequent BOP/HOBt/DIEA mediated head-to-tail cyclisation. Interestingly, the strategy where a deprotected C-terminal aspartyl residue (Asn⁸ constituted by coupling of Fmoc-Asp to a PAL handle resin) was coupled to the deprotected Nα of a N-terminal aspartyl residue, in the absence of Fmoc protection group, was found to work best as compared to other synthetic strategies obtained by permutation of the starting point of synthesis along the cyclic peptide sequence.

A limiting disadvantage of this method is that it is tacitly taken into account that partial Fmoc deprotection occurs as a side reaction during the main allyl deprotection step, due to the presence of nucleophilic reagents and because it does not truly affect the reaction scheme. Further completion of Fmoc deprotection takes place subsequently in any event, and is required to allow subsequent head-to-tail peptide bonding. In contrast, cyclisation by means of lactamization of peptide side chain functionalities only is crucially dependent on preserving complete protection of the Nα.

Blankemeyer et al. (1988, Tetrahedron Lett. 29, 5871-5874) described the synthesis of several protected peptide fragments on cellulose discs containing 4-(4'-methoxytrityloxy)-but-2-enyloxyhexanoic acid as an allylic handle to the solid phase, further employing Fmoc chemistry. Cleavage of the allyl ester linker was achieved by treatment with Pd(PPh₃)₄ in dry THF followed by the addition of a solution of HOBt in THF.

Another method of peptide side chain cyclisation is devised according to the present invention, avoiding the disadvantages of the prior art and being particulary useful for cyclisation of peptides comprising aspartyl side chains.

According to the present invention, a cyclisation method for a peptide comprises the steps of
(a) deprotecting the peptide from allyl-type protecting groups, which peptide is protected with a base-labile protection group at its Nα and which peptide further comprises at least one allyloxycarbonyl-protected amino function of a lysine side chain (i.e. the ε-amino group) or of an analogue of such lysine side chain and further comprises at least one allylester-protected ω-carboxyl group of a glutamyl (i.e. a β-carboxy group) or aspartyl side chain (i.e. a γ-carboxy group) or of an analogue of such side chain whilst retaining the base-labile protection group on the Nα,
(b) cyclising the peptide by lactamisation of said deprotected side chains in the presence of a weak base reagent, and
(c) deprotecting the peptide from the base-labile protection group at its Nα,
wherein said allylic protection groups may be unsubstituted or may be further substituted with alkyl or aralkyl that in itself may be unsubstituted or further substituted with halogen or alkoxy. In a preferred routine embodiment, the standard unsubstituted Alloc (i.e. allyloxcarbonyl) protection group is employed for protection of the ε-amino-function and the γ-carboxy group is protected by esterification with allyloxy.

In the present context, the 'ω-carboxyl group' of an amino acid side chain is understood as being the 'terminal carboxyl group of a side chain irrespective of the carbon chain length, and the 'ω-amino group' of an amino acid side chain is understood as being the 'terminal' amino group of a side chain irrespective of the carbon chain length. Analogues thereof may well be e.g. side chain isomers thereof. For example, a γ- or δ-amino isomer of lysine is a suitable analogue of natural lysine. In the context of the present invention, the term 'side chain' in respect to an amino acid or amino acid derivative is used in compliance with the respective IUPAC-IUB definition (International Union of Pure and Applied Chemistry and International Union of Biochemistry/Joint Commission on Biochemical Nomenclature, "Nomenclature and Symbolism for Amino Acids and Peptides", Pure Appl. Chem, 56, 595-624 (1984)).

This reaction path has not been described before. Unexpectedly, it has been found that integrity of the Fmoc group is largely preserved after allyl-deprotection, in particular when allyl-deprotection is carried out on solid-phase bound peptide, and in consequence cyclisation is also carried out on the solid-phase bound material. Preferably, after completion of the reaction sequence, peptide chain elongation is continued, more preferably is continued in a solid-phase mode, since having the advantage that the preceding cyclisation step would then not require application of limiting dilution conditions for favoring intramolecular cyclisation over intermolecular reactions. The present reaction will hence be particulary beneficial where multiple glutamyl and/or aspartyl and lysine residues or their analogues such as e.g. nor- or homo-lysine, are present in the final peptide chain whilst only a specific pairing of those residues is scheduled for cyclisation on a firstly synthesized portion of the final full-size peptide chain. This regional-lactamisation approach will be particularly beneficial when the full-size peptide comprises several subsequent subdomains or peptide loop structures for bioactive peptides that are to be stabilized by side-chain cyclisation. Lactamization is a more stable, less redox-vulnerable option than naturally stabilized disulfide-bridging or non-natural, chemical analogues thereof employing amino-acid analogues. The latter functionalities usually prove to be more immunogenic in vivo, whereas the present invention allows use of natural amino acid analogues as a superior option.

In a preferred embodiment, the peptide contains just one allyl-protected lysine residue and just one allyl-protected aspartyl or glutamyl residue, and hence there is one and only one bonding option upon cyclisation, giving rise to a homogenous product.

In contrast to prior art methods, the peptide according to the method of the present invention can be utilized for further N-terminal peptide elongation, optionally and preferably while on solid phase, be it in the sequential, stepwise mode of adding or modifying amino acid residues or other groups, such as by addition of new, N-protected amino acids or be it by N-terminal condensation reaction with another peptide.

In a further particularly preferred embodiment, the N-terminal residue of the protected peptide having a base-labile protection group at its Nα is said at least one allyl ester-protected aspartyl or glutamyl residue or analogue thereof. A seldom appreciated problem with such N-terminally protected, preferably Fmoc protected, and simultaneously side chain-protected amino acids is that they are prone to a base-catalyzed side reaction upon Fmoc deprotection, giving rise to either aspartimide or glutarimide. It is an undesired shortcoming of present standard solid phase chain elongation synthesis employing FMOC protection groups that is usually believed to be avoided by protection of γ-carboxylic acid groups. However, this is not true as has been shown by the reports of Kates et al. and others (Kates et al., Lett. Pept. Sci., 1995, 1, 213; Nicolas et al., Tetrahedron Lett. 1989, 30, 497) for different protection groups for γ-carboxy functionalities. The same holds true according to the present inventors' observation for allyl ester protection of the γ-carboxy functionality of an Fmoc protected, N-terminal aspartyl residue under standard solid phase conditions for Fmoc deprotection. Its precise quantitative extent is highly sensitive to slight changes in processing time and conditions, giving rise to undesired variation of product yield:

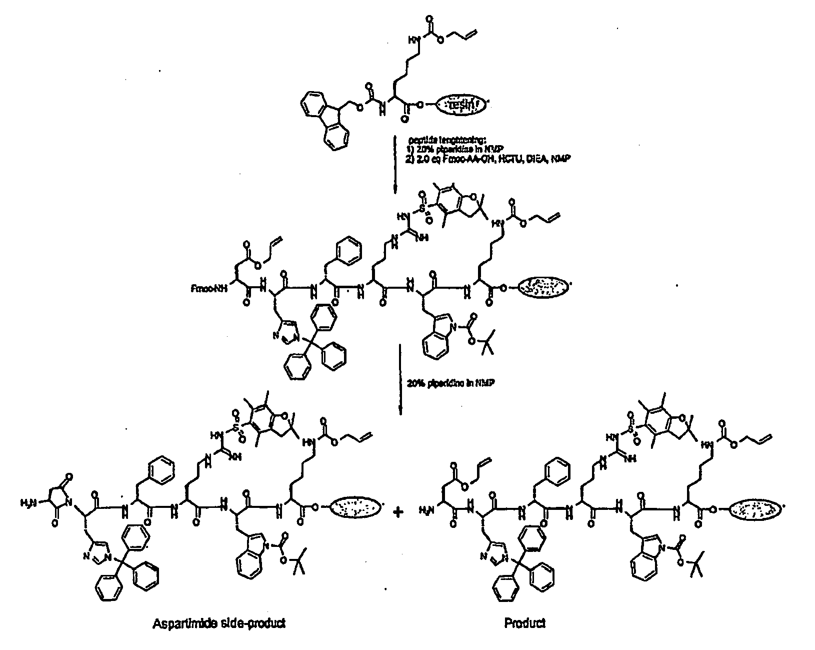

Accordingly, with the method of the present invention, it is now possible to avoid this unproductive side reaction by immediate side chain cyclisation of such N-terminal, Fmoc and allyl ester-protected aspartyl or glutamyl residue. Again, Fmoc protection is preserved, effectively preventing aspartimide formation. Thus, subsequent Fmoc deprotection followed by Further chain elongation is possible without glutarimide or aspartimide formation due to the prior lactamization.

More preferably, the N-terminal allylester-protected acidic residue is an aspartyl residue. Aspartimide formation has a higher reaction rate than glutarimide formation and is usually the more dominant side reaction. It is particularly favored in the dipeptide sequence L-Asp-L-X where X is Gly, Ser, Thr or Asn, and as we report here for the first time, where X is His, easily yielding up to 30 or 40% aspartimide. In particular the Asp-Gly- dipeptide is highly prone to aspartimide formation; usually, this requires use of an additional HMB protection group on the glycine, providing steric hindrance of aspartimide formation as described in Packman et al., 1995, Tetrahedron Lett. 36, 7523. However use of Gly(HMB) dipeptides is extremly expensive and hence is sought to be avoided. Thus in these instances the present invention offers even a further advantage over the prior art.

The allyl and/or alloc deprotection step (in the following, allyl deprotection for short) may be carried out by the methods known in the art, such as e.g. hydrostannolysis with Bu₃SnH or treatment with tetrakis(triphenylphosphine)palladium(0) [Pd(PPh₃)₄] in THF at essentially neutral conditions in the presence of a nucleophile such as e.g. morpholine, dimedone, N-methylaniline, HOBt, borohydride or N,N'-dimethylbarbituric acid as an allyl acceptor. Variants of said methods employing different pH exist, but of course care must be paid in view of the pH sensitivity of resin linkage or handle groups and the base sensitive protection group.

The allyl deprotection preferably employs catalysis with allyl-group reactive palladium complexes, preferably with Pd(0) complexes, preferably with palladium complexes having C₁-C₁₀ trialkyl phosphite, C₃-C₁₀ tricycloalkyl phosphite, triarylphosphine or triheteroarylphosphine ligands, wherein said aryl or heteroaryl may be further substituted with electron-donating substitutents or is unsubstituted, more preferably with palladium complexes having phenylphosphine ligands wherein the phenyl may be further substituted with C₁-C₅ alkyl, preferably wherein the phenyl is tolyl or xylyl, more preferably is phenyl, 2,4-xylyl or o-tolyl. Preferably, said phosphine ligands are mono-phosphine ligands, more preferably non-chelating, monovalent ligands. Although the palladium complexes preferably are mono-palladium complexes, the term complex is to be understood as to also comprise di-palladium or higher palladium complexes, though mono-palladium complexes are preferred. Using tetrakis(triphenylphosphine)/palladium [Pd(PPh₃)_{4]}, in the presence of an allyl acceptor or scavenger, or using corresponding Pd (P[o-tolyl]₃)₂ or Pd(P[2,4-xylyl]₃)₂ complexes or mixed complexes possibly having both triphenylphosphine and tri(o-tolyl)phosphine ligands is mostly preferred in the present invention. As compared to triphenylphosphine, methylphenylphosphine ligands and especially the tri(o-tolyl)phosphine ligands improve the catalytic reaction rate, further allowing lowering the total amount of precious metal catalyst used whilst maintaining optimal yields. For reference to Pd(0)-catalyzed allyl and allyloxy deprotection, compare Jeffrey et al., J. Org. Chem. 1982, 47:587-590. As has been described in Jeffrey, it is also feasible and perfectly customary in the art to constitute the complexes of the present invention as an exchange catalyst *in situ,* namely by mixing less stably coordinated Pd-complexes with the preferred ligands of the present invention. Hence examples of suitable catalyst complexes apart from the strongly preferred Pd(PPh₃)₄ are: PdCl₂(PPh₃)₂ / PPh₃, PdCl₂(PPh₃)₂ / P(o-Tol)₃, Pd(DBA)₂ / P(o-Tol)₃ or Pd[P(o-Tol)₃]₂ (Organometallics 1995, 14(6):3030-3039), Pd(OAc)₂ /triethylphosphite, Pd(OAc)₂ / PPh₃ or Pd(OAc)₂ P(o-Tol)₃. The presence of less coordinating anionic, basic ligands such as acetate/ or amines (e.g. Huenig base) in the initially added Pd(0) complexes allows constituting the preferred complexes *in situ* due to the presence of suitable free ligands such PPh₃ or P(o-Tol)₃ [o-Tol = ortho-tolyl]. It is not mandatory in general to add free ligand for exchange with the catalyst though, especially when using highly active arylic phosphine palladium complexes from the start - this option is noteworthy in case of zerovalent Pd(PPh₃)₄ which was originally used by Jeffrey et al. (supra) with extra PPh₃ added to the reaction broth. The general mechanism of Pd-catalyzed deprotection in the presence of an allyl acceptor is an acyl group transfer reaction (transacylation), as is well known in the art. Hence the choice of allyl acceptor reagent or scavenger is likewise important for achieving quantitative deprotection under mild reaction conditions whilst avoiding unwanted side reactions. A suitable scavenger is any nucleophile such as e.g. morpholine, dimedone, N,N-dimethylbarbituric acid, methylaniline or thiosalicylic acid.

Suitable catalytic amounts of Pd(0) complexes preferably are used in an amount of 0.005 eq. to 0.5 eq. catalyst as compared to educt, more preferably are used in an amount of 0.01 up to 0.1 eq. of catalyst, most preferably are used in an amount of 0.015 eq. up to 0.07 eq. of catalyst. Preferably, reaction temperature is in between 10-60°C, more preferably in between 30-50°C, most preferably at about 40°C.

In one preferred embodiment of the present invention, amine-borane complexes are employed in at least 1.5 to 2-fold excess per allyl function of the educt as the nucleophilic allyl group scavenger as described in Gomez-Martin et al., J. Chem. Soc., Perkin Trans. 1(1999): 2871-2874. Depending on the exact composition of the amine moiety in the complex, high conversion rates along with very short reaction time can be realized, for instance with t-Bu-NH₂·BH₃, Me₂NH·BH₃ or NH₃·BH₃. Quaternary amines are excluded from the present definition of suitable complexes, whereas the amine may be preferably a primary or secondary alkyl amine or may be ammonia. In an optional, even more preferred embodiment, said Pd(0) catalyzed allyl-deprotection is carried out as a hydrosilylolysis in the presence of the hydride donor phenyl-trihydrosilane PhSiH₃ or functional derivatives thereof in an aprotic, polar organic solvent such as e.g. dichloromethane, as has been essentially described by Dessolin et al., Tetrahedron Lett. 1995, 36: 5741-5744. Preferably, a phenyl-hydrosilane reagent of the generic formula (R1-Ph)ₙSiH₄₋ₙ or PhSiH₃ is employed as the allyl acceptor that is usually in excess of at least 1.5 to 2 eq. wherein R1 is a substituent at the aromatic core and is aryl, alkyl or aralkyl, and n is 1 or 2. Most preferably the allyl acceptor is PhSiH₃.

Both phenylsilanes and suitable amine-borane complexes allow of rapid, complete deprotection in the range of < 1h, typically at about 20-40 min. Accordingly, they allow mild and short reaction conditions. Other allyl scavengers may require considerable longer reaction times.

Though technically perfectly feasible reagents, it must be mentioned that both classes of above allyl scavengers are noxious reagents that may raise concerns from an environmental and working safety point of view. In another strongly preferred embodiment that is particularly attractive for industrial scale manufacture, an organic sulfinate is used as an allyl group acceptor reagent (Honda et al., J. Org. Chem. 1997, 62, 8932-8936). Surprisingly, in the reaction of the present inventions, this reaction allows particular high product yields, possibly due to the stability of the adducts thus formed and the mild reaction conditions. Examples of such are 4-chloro-3-nitrobenzenesulfinate, 2-thiophenesulfinate, benzenesulfinate, p-toluenesulfinate or hydroxymethanosulfinate (also known in form of its sodium or zinc salt as Rongalit™, the traditional trivial name for such salts having been formaldehyde sulfoxylates). The organic sulfinate Rᵢ-SO₂⁻ may comprise any type of further substituted organic residue, like alkyl, cylcoalkyl, aryl, heteroaryl or aralkyl (cf. Honda et aL, supra). It may be added in its salt or acid forms. Even the presence of competing nucleophilic moieties in said sulfinate is possible, though less preferred. Preferably, for optimal yields attainable, the radical Ri is an optionally further substituted phenyl radical, more preferably is a single or multiple, alkyl-, alkyloxyalkyl- or alkyloxy-substituted phenyl radical, and most preferably is phenyl, xylyl or tolyl, especially p-tolyl. Use of sulfinates also allows use of trialkyl or cycloalkyl phosphite ligand complexes with good yields, in addition to the more preferred triarylic phosphine complexes.

The lactamisation reaction is carried out in an essentially similiar manner as standard peptide chain elongation reactions in the presence of base-labile amine-protection groups such as Fmoc, except that the base labile group is carried on the same peptide rather than adding a further N-terminally protected amino acid as in chain elongation. Of course, the same chemistry may subsequently be employed for further chain elongation in an optional step d. after cyclisation and deprotection. Both lactamization and chain elongation require a coupling reagent and eventually a coupling additive, depending on the type of primary coupling reagent or auxiliary.

Coupling reagents for peptide synthesis are well-known in the art (see Bodansky, M. , Principles of Peptide Synthesis, 2nd ed. Springer Verlag Berlin/Heidelberg, 1993; also see discussion of role of coupling additives or auxiliaries therein). Coupling reagents may be anhydrides (e.g. T3P: propanephosphonic anhydride) or other acylating agents such as activated esters or acid halogenides (e.g. ICBF, isobutyl chloroformate), or they may be carbodiimides (e.g. 1-ethyl-3-(3-dimethylaminopropyl)-carbodiimide), activated benzotriazine-derivatives (DEPBT: 3-(diethoxyphosphoryloxy)-1,2,3-benzotriazine-4(3H)-one) or uronium or phosphonium salt derivatives of benzotriazole.

In view of the best yield, short reaction time and protection against racemization during chain elongation, more preferred is that the coupling reagent is selected from the group consisting of uronium salts and phosphonium salts of benzotriazole capable of activating a free carboxylic acid function where the reaction is carried out in the presence of a base. Suitable and likewise preferred examples of such uronium or phosphonium coupling salts are e.g. HBTU (O-(1H-benzotriazol-1-yl)-N,N,N',N'-tetramethyluronium hexafluorophosphate), BOP (benzotriazol-1-yloxy-tris(dimethylamino)phosphonium hexafluorophosphate), PyBOP (benzotriazol-1-yloxy-tripyrrolidinophosphonium hexafluorophosphate), PyAOP, HCTU (O-(1H-6-chlorobenzotriazol-1-yl)-1,1,3,3-tetramethyluronimn hexafluorophosphate), TCTU (O-(1H-6-chlorobenzotriazol-1-yl)-tetramethyluronium hexafluorophosphate), TCTU (O-(1H-6-chlorobenzotriazol-yl)-1,1,3,3-tetramethylumnium tetrafluoroborate), HATU (O-(7-azabenzotriazol-1-yl)-1,1,3,3-tetramethyluronium hexafluorophosphate), TATU (O-(7-azabenzotriazol-1-yl)-1,1,3,3-tetramethyluronium tetrafluoroborate), TOTU (O-[cyano(ethoxycarbonyl)methyleneamino]-N,N;N',N'-tetramethyluronium tetrafluoroborate), HAPyU (O-(benzotriazol-1-yl)oxydipyrrolidinouronium hexafluorophosphate.

Preferably, the base reagent is a weak base whose conjugated acid has a pKa value of from pKa 7.5 to 15, more preferably of from pKa 7.5 to 10, with the exclusion of an α-amino function of a peptide or amino acid or amino acid derivative, and which base preferably is a tertiary, sterically hindered amine. Examples of such and further preferred are Hünigbase (N,N-diisopropylethylamine), N,N-dialkylaniline, 2,4,6-triallcylpyridine, 2,6-dialkylpyridine or N-alkylmorpholine with the alkyl being straight or branched C₁-C₄ alkyl, more preferably it is N-methylmorpholine or collidine (2,4,6-trimethylpyridine), most preferably it is collidine.

The use of coupling additives, in particular of coupling additives of the benzotriazole type, is also known (see Bodansky, supra). Their use is particularly preferred when employing highly activating uronium or phosphonium salt coupling reagents. Hence it is further preferred that the coupling reagent additive is a nucleophilic hydroxy compound capable of forming activated esters, more preferably having an acidic, nucleophilic N-hydroxy function wherein N is imide or is N-acyl or N-aryl substituted triazeno, most preferably the coupling additive is a N-hydroxybenzotriazole derivative (or 1-hydroxybenzotriazole derivative) or is an N-hydroxybenzotriazine derivative. Such coupling additive N-hydroxy compounds have been described in WO 94/07910 and EP-410 182 and the respective disclosure is incorporated by reference hereto. Examples are e.g. N-hydroxysuccinimide, N-hydroxy-3,4-dihydro-4-oxo-1,2,3-benzotriazine (HOOBt), 1-hydroxy-7-azabenzotriazole (HOAt) and N-hydroxybenzotriazole (HOHt). N-hydroxybenzotriazine derivatives are particularly preferred, in a most preferred embodiment, the coupling reagent additive is N-hydroxy-3,4-dihydro-4-oxo-1,2,3-benzotriazine. Ammonium salt compounds of coupling additives are known and their use in coupling chemistry has been described, for instance in US 4,806,641.

In a further particularly preferred embodiment, the uronium or phosphonium salt coupling reagent is an uronium salt reagent and preferably is HCTU, TCTU or HBTU and even more preferably is used in the reaction in combination with N-hydroxy-3,4-dihydro-4-oxo-1,2,3-benzotriazine or a salt thereof. This embodiment is mainly preferred for use in chain elongation step of peptide synthesis after removal of the base-labile Nα-protection group, but may as well be used for lactamization reaction during side-chain cyclisation.

In the context of the present invention, it is to be noted that HCTU and TCTU are defined as to be encompassed by the term 'uronium salt reagent' despite that these compounds and possible analogues have been shown to comprise an isonitroso moiety rather than an uronium moiety by means of crystal structure analysis (O. Marder, Y. Shvo, and F. Albericio "HCTU and TCTU: New Coupling Reagents: Development and Industrial Applications", Poster, Presentation Gordon Conference February 2002), an N-amidino substituent on the heterocyclic core giving rise to a guanidium structure instead. In the present context, such class of compounds is termed the 'guanidium-type subclass' of uronium salt reagents according to the present invention.

In a further particularly preferred embodiment, which is mainly used for the lactamization reaction, the coupling reagent is a phosphonium salt of the benzotriazol such as e.g. BOP, PyBOP or PyAOP.

Deprotection of the base labile Nα may be carried out as routinely done in the art, e.g. with 20% piperidine in N-methylmorpholine.

A further object of the present invention is a cyclic peptide of formula II or III having an Nα that is protected with a base-labile protection group, 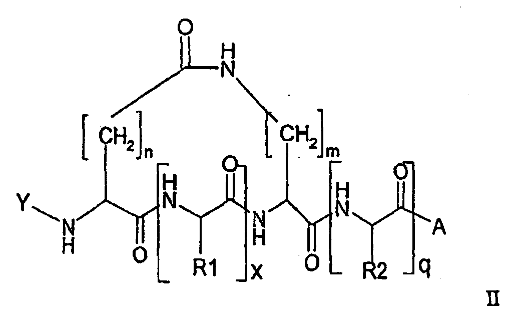 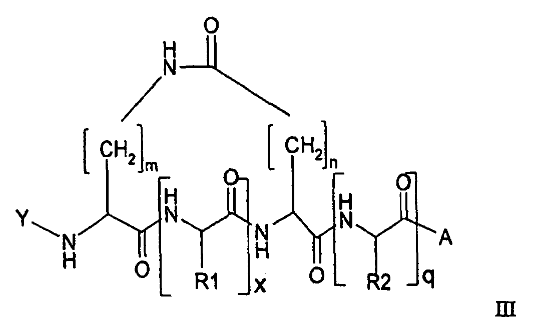 wherein Y is the base-labile protection group, n = 1-10, preferably n = 1 or 2, most preferably n = 1, further wherein m = 1-15, preferably m = 3 to 6, most preferably m = 3, further wherein x = 1-200 and q = 0-200 wherein R1 and R2 each are, independently, a natural amino side chain or non-natural derivative thereof, which side chain further may comprise a protection group with the exception of allyl ether and allyloxycarbonyl protection groups, and wherein A is a resin or resin handle; or wherein R1 is as defined above and R2 is a natural amino side chain or non-natural derivative thereof which side chain is bonded to a resin or resin handle via an ether, thioether, ester, thioester, amido or secondary or tertiary amino moiety and A is selected from the group consisting of OH, NH₂, NR1H or NR1'NR2', OR3 with R1' and R2' being independently C₁-C₄ alkyl and R3' being a protection group with the exception of allyl groups, preferably with R3' being tert-butyl or pentafluorophenyl.

A side chain group, such as for example R1₍ₓ₌₁₎, is not to be construed to refer to a single type of optionally protected amino acid side chain; that is, each residue R1(1), R1(2), and so on may be unique or may be the same as at least one other residue. The same applies of course to radicals R2_{(q=1)}, R2_{(q=2)}, and so on.

The resin or resin handle composite entity may in principle be any resin employed for synthesis, such as for example a polystyrene-divinylbenzene resin as used by Merrifield along with hydroxybenzyl-phenyl integral linker moieties or by Wang with hydroxybenzyl-p-benzyloxy moieties, such as for example moieties to which e.g. more acid-labile linkers may be further grafted, or alternatively the latter linkers may be integrally or directly linked to the resin. In principle, a solid phase resin for use in synthesis necessarily comprises at least an integral linker or handle which is part of the solid phase core material; such linker or handle may be considered as an immobilized protection group (Guillier et al., Chem. Rev. 100, 2091-2157, 2000). Examples are e.g. Sieber resin, related xanthenyl type PAL handle resins, Rink amide resin, Rink acid resin, more complex PEG-grafted polystyrene resins such as tentagel-based Novasyn TG (Novabiochem, Merck Biosciences, Germany) which are available with different grafted handles such as 2-chlorotrityl, or resins that are constituted by grafting functional handles onto matrix material such as silica gels. Preferably, where the resin is a trityl resin or resin handle, such resin is a 4-methoxy- or 4,4'-dimethoxytrityl resin. Resins as used in the present invention are of standard mesh size, which is about 50-500 mesh, more preferably 100 to 400 mesh. A resin or solid-phase R"' as shown in formula IV is to be construed as to comprise a crosslinked, polymeric matrix material which may be bound to the handle moiety specified in formulas IV to VII by way of any kind of chemically inert alkyl, alkyloxy, aryloxy or alkyl ester spacer or linker which is to be considered an integral part of R"'. However, it should be noted that apart from impacting the conditions of cleavage from the resin, the chemical nature of the resin material and in particular the chemical nature of the handle group may well influence synthetic efficiency of coupling and especially lactamisation reactions in a yet poorly understood fashion. The yields of mature peptide at the on-resin stage may differ depending on the type of resin or resin handle employed. For this reason, in an preferred embodiment according to the present invention the resin or resin handle is of formula IV as set forth in the claims in detail, more preferably of formula VI and most preferably of formula VII as set forth in the claims in detail. Examples of such resins or resin handles are (4-methoxyphenyl)-methyl- and (4-methylphenyl)-methyl-polystyrene (Atkinson et al., 2000, J. Org. Chem. 65, 5048), resins in O- or N-linkage to the peptide moiety and their PEG-resin derivatives, respectively. Further examples are e.g. acid-labile HMPB-MBHA or HWPB-BHA resin (Sieber et al., 1987, Tetrahedron Lett. 28, 6147), acid-labile Rink amide resin or Rink acid resin (Rink et al., 1987, Tetrahedron Lett. 28, 3787). The term 'acid-labile' refers to essentially quantitative cleavage in 2-10% TFA in dichloromethane at ambient temperature for at least an hour. Surprisingly, using such preferred resins having the diphenyl-methyl structural core motif allow for more efficient coupling reaction during linear synthesis and lactamisation; notably, such resins also allow a lower reaction temperature of 15-25°C as compared to the standard 40°C required for efficient coupling on e.g. trityl resins.

Preferably radical A (as given e.g. in formula II or III) comprises a resin handle or resin linkage moiety with the exception of resin handles comprising an allyloxycarbonyl moiety. More preferably, such resin or resin handle is of formula IV 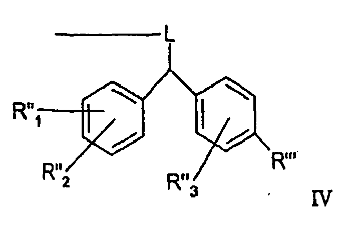 wherein R"' is a resin, and R1", R2", R3" are, independently, hydrogen, C₁-C₄ alkyl or C₁-C₄ alkoxy, and may be the same or different with the proviso that only one of R1", R2" may be hydrogen, and wherein L is oxygen, sulfur, nitrogen or is of formula V

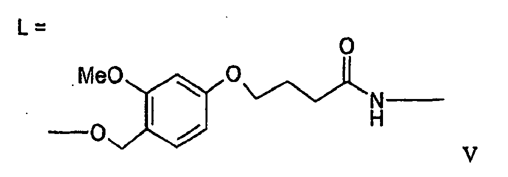

Again even more preferred is that resin or resin handle is of formula VI, the above definitions for radicals R"', R1" and R2" applying with the proviso that L is selected from the group consisting of oxygen and nitrogen,

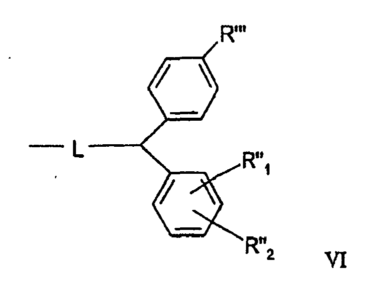

Again even more preferred is that the resin or resin handle is of formula VII, the above definitions for radicals R"', R1" and R2" applying with the proviso that L is selected from the group consisting of oxygen and nitrogen,

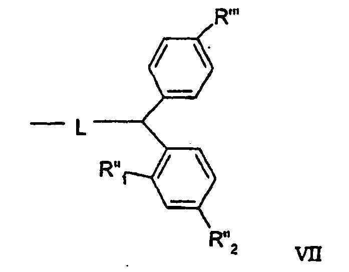

In a further even more preferred embodiment, it is preferred that R1", R2" are, where L is oxygen, independently hydrogen, methyl or methoxy with the proviso that only one of R1", R2" may be hydrogen, and where L is nitrogen, independently are methyl or methoxy, preferably are methoxy. Even more preferably then, L is oxygen, R1" is hydrogen and R2" is methyl or methoxy. Most preferably, R2" is methyl.

Most preferably, the peptide according to the present invention is carboxy terminally coupled to the resin or resin handle (A = resin or resin handle in formula IV).

Preferably, the peptide sequence according to the present invention is Ac-Nle-cyclo(Asp-His-D-Phe-Arg-Trp-Lys) or NIe-cyclo(Asp-His-D-Phe-Arg-Trp-Lys), a lactam bond being in place between the Asp and Lys side chains as shown in scheme 1. Said peptide is a pharmaceutically active melanocortin receptor-specific peptide useful for treatment of sexual dysfunction, including male erectile dysfunction and female sexual dysfunction in humans.

In another preferred embodiment, the peptide sequence according to the present invention consists of or comprises at least the partial sequence cyclo(Asp-His-Phe.Arg-Trp-Lys), wherein the Phe residue may also be D-Phe, or the respective D-or L-isomer of Phe(4-7), Phe(4-Br), Phe(4-CF₃), Phe(4-CI), Phe(2,4-diCl), Phe(3,4-diCl), Phe(3,4-diF), Phe(4-I), Phe(3,4-di-OMe), Phe(4-Me) or Phe(4-NO₂). These modifications with non-natural derivatives of Phe modulate pharmaceutical activity of said peptide. Likewise, in the above sequence, the Arg may also be D-Arg, or the respective D- or L-isomer of Arg(NO₂), Arg(Tos), Arg(Pbf), Arg(Mtr), Arg(Me) or Arg(Pmc).

The arginine side chain may be preferably covalently protected during synthesis e.g. with tosyl (Tos), benzyloxycarbonyl, pentamethylchromanesulfonyl (Pmc), pentamethyldihydrobenzofuranesulfonyl (Pbf), 4-methoxy-2,3,6-trimethylbenzenesulfonyl (Mtr) and its 4 tBu-2,3,5,6-tetramethyl homologue (Tart), adamantyloxycarbonyl or Boc. Pmc, Pbf, Mtr or Tart are strongly preferred for protecting Arg, most preferably it is Pbf.

Trp is preferably protected during synthesis with Boc. Optionally, it may be N-protected with formyl or mesitylenesulfonyl.

His is preferably protected by N-trityl protection group. Optionally, though less preferred, it may be likewise N-protected with Boc, methyltrityl or tosyl.

### Experiments

A fully protected Fmoc version of the peptide Ac-Nle-*cyclo*(Asp-His-D-Phe-Arg-Trp-Lys) - whose properties were described in WO 01/000224 drawn prominently to said peptide only - was newly synthesized on different resins, e.g. on 2-chlorotrityl-polystyrene resin, and processed according to the present invention as shown in scheme 1. N-terminally, in the first step of the reaction scheme shown in scheme 1, the sequence is Fmoc Asp(All)-His(Trt)-.... The Lys(Alloc) and the Asp(All) are five residues spaced apart, with bulky side chains such as e.g. Arg(Pbf) protruding in between. In the scheme below, Nle is norleucine.

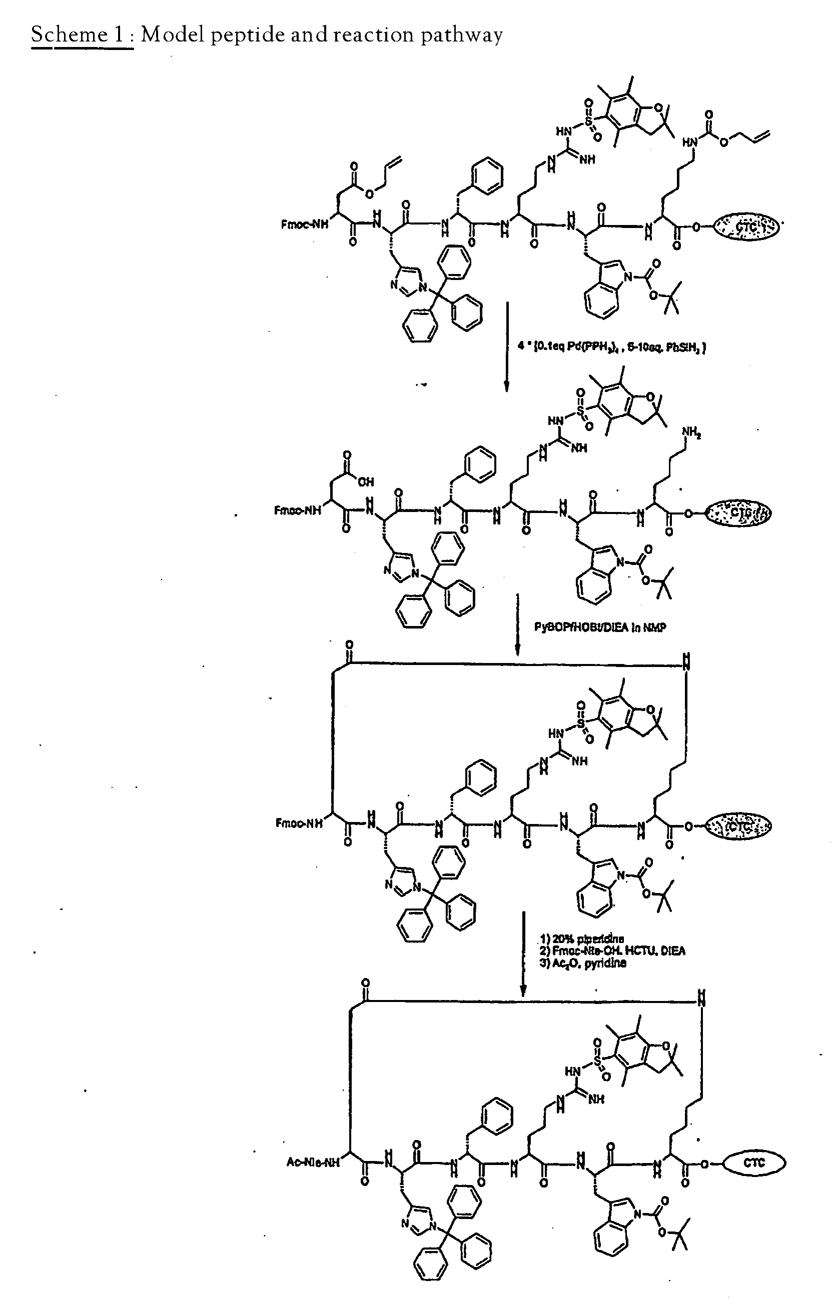

### 1.1 Allyl/alloc deprotection of 2-CTC resin conjugated model peptide

0.1 eq. of Pd(PPh₃)₄ was solubilized in DCM in the presence of PhSiH₃ or diaminoborane (5 eq.).
This solution was added to the CTC-resin carrying 1 eq. (35 g resin, loaded at 0.44 mmol/g) of the allyl/alloc protected starting peptide of scheme 1. After max. 15 min of reaction at room temperature under steady nitrogen bubbling, the mixture was filtered, and the recovered resin subjected three more times to the same treatment, always with the same amount of catalyst and 5 eq. of phenylsilane as scavenger per cycle.

Washing step: Afterwards the resin was consecutively washed with
• N-methylpyrrolidone (NMP) (3 times)
• Dichloromethane (DCM) (3 times)
• 0.5% DIEA (Hünig base) in DCM (3 times)
• Sodium diethyldithiocarbamate trihydrate 0.02 N in NMP
• NMP (5 times)

### 1.2 Cyclisation: Lactamisation of side chains

1 eq. of PyBOP and 1.8 eq. of HOBt were solubilized in NMP and added to the Asp / Lys-unprotected Fmoc peptide in the presence of 4 eq. of DIEA. The reaction was stirred for 2 hours at room temperature. The lactamized peptide was recovered by filtration and washed with NMP.

HPLC reverse phase analysis (C-18 column, loading of about 0.1 g/ml solution in 0.1% TFA 70% water/30% acetonitrile, elution with 0.1% TFA acetonitrile gradient) of the intermediate peptide samples released with 2% TFA from the CTC resin showed that conversion was almost complete and that >90% of the lactamized, protected peptide retained the Fmoc moiety.

### 1.3 Fmoc deprotection and peptide elongation

Fmoc deprotection was carried out with 20% piperidine in NMP. Subsequently, chain elongation was carried out for 30 min. in DMF at about room temperature (40°C) for 1 h with Fmoc-L-Nle (leq.) in the presence of 1 eq. HCTU and 3 eq. HOOBt, 3 eq. DIEA.
After filtering off the resin-bound product and washing with DMF, the Fmoc moiety on Nle was removed with 20% piperidine in NMP, and an N-terminal acetyl group incorporated by incubation in pyridine with about 1.5 eq. of acetic anhydride for 1-2 h at room temperature.

After filtering off the resin, the peptide was released and globally deprotected by concentrated TFA treatment. The total yield of acetylated, lactamized peptide based on crudely purified product was roughly determined to amount to ~60%.

The need for further, final on-resin derivatization with an acylating reagent further demonstrates the utility of the present invention, namely to carry out lactamization in the presence of, but without compromising a base-labile No-protection group. Using acid-cleavable N-protection groups such as Boc would have created further problems here at least for on-resin processing.

### 2.1 Allyl/alloc deprotection of bromo-(4-methylphenyl)methyl-polystyrene resin conjugated model peptide in the presence of an aminoborane.

Experiment 1.1 was repeated, using (CH₃)₂NH·BH₃ as scavenger and exchanging the CTC resin for bromo-(4-methylphenyl)methyl-polystyrene (approx. 200 mesh, 1.2-2.2 mmol/g) (CBL Patras, Greece). Catalyst was solubilized in AcOH/DMF (approx. 4:1), whilst deprotection reaction was carried out in DMF (5 times resin volume) at 40°C for 30 min. Steps 2.2 and 2.3 were carried out as described in chapters 1.2 and 1.3 above, except that cleavage was conducted in 5% TFA in dichloromethane in the presence of 2% TIS. Yield of acetylated, mature peptide amounted to 72.8% (analytical grade purity).

### 3.1 Allyl/alloc deprotection of bromo-(4-methylphenyl)methyl-polystyrene resin conjugated model peptide with exchange catalyst Pd(OAc)₂/P(o-Tol)₃

Reaction 2.1 was repeated, with the following modifications: Instead of 0.1 eq. of Pd(PPh₃)₄, 0.05 eq. of Pd(OAc)₂ in the presence of 0.05 eq. of tri(toly)phosphine were used. Further, 2.2 eq. of sodium p-toluenesulfinate were added as scavenger. Even after 2 hours, conversion had only taken place in trace amounts; raising the temperature to 60°C did not change that.

### 4.1 Ally/alloc deprotection of bromo-(4-methylphenyl)methyl-polystyrene resin conjugated model peptide with exchange catalyst Pd(P[o-Tol]₃)₂ and with sulfinate

Catalyst Pd(P[o-Tol]₃)₂ was obtained as described in Paul et al., Organometallics 1995, 14(6), 3030-3039. Catalyst was solubilized in AcOH/DMF(2:1). The reaction was carried out as described in chapter 2.1, but with using 0.05 eq. of Pd(P[o-Tol]₃)₂ and 2.2 eq. of sodium p-toluenesulfinate were added as scavenger under steady nitrogen bubbling. Reaction took place for 30 min. at 40°C. The reaction proceeded smoothly, with the analytical yield of cleaved, acetylated peptide amounting to 82% after step 4.2, step 4.3 having been carried out as in section 2 above. Extending the reaction time of allyl/alloc deprotection to 150.min. did not significantly increase yield (yield: 84.5%). Lowering the amount of catalyst by about half slowed the reaction kinetic considerably.

### 5.1 Allyl/alloc deprotection of bromo-(4-methylphenyl)methyl-polystyrene resin conjugated model peptide with exchange catalyst Pd(PPh₃)₄ and with sulfinate

Reaction 4.1 was repeated as described above, except that now 0.1 eq. of Pd(PPh₃)₄ were used as the catalyst and 30-60 min. reaction time was employed for allyl/alloc deprotection. The yield of cleaved, acetylated mature peptide amounted to 82%.

The preceding examples can be repeated with similar success by substituting the generically or specifically described reactants and/or operating conditions of this invention for those used in the preceding examples.

## Claims

1. Method of cyclisation of a peptide by lactamisation, comprising the steps of
(a) deprotecting the peptide from allyl-type protecting groups by Pd(0) catalysis in the presence of an allyl acceptor, which peptide is protected with a base-labile protecting group at its Nα and further comprises at least one allyloxycarbonyl-protected amino function of a lysine side chain or of an analogue of such lysine side chain and further comprises at least one allyl ester-protected ω-carboxyl group of a glutamyl or aspartyl side chain or of an analogue of such side chain whilst retaining the base-labile protection group on the Nα, wherein said allylic protecting groups are substituted or unsubstituted,
(b) cyclising the peptide by lactamisation of said deprotected side chains in the presence of a weak base reagent, and
(c) deprotecting the peptide from the base-labile protection group at its Nα.

2. Method according to claim 1, **characterized in that** the Nα protected with a base labile protection group is the Nα of an aspartyl residue having said further allylester-protected ω-carboxyl group.

3. Method according to claim 1 or 2, wherein the aspartyl residue is part of the dipeptide sequence Asp-His.

4. Method according to claim 1, 2 or 3, **characterized in that** it comprises as a further step(d), further elongating the peptide by means of continued peptide synthesis or peptide segment coupling of the deprotected Nα.

5. Method according to claim 4, **characterized in that** the peptide is bound to a solid phase during cyclisation, with the proviso that the allylic protection groups are not serving as a resin handle, and that the peptide elongation of step (d) is a continued solid phase peptide synthesis.

6. Method according to claim 1, 2 or 3, **characterized in that** the cyclisation reaction step b. is carried out in the presence of a weak base reagent in an aprotic polar solvent and in the further presence of a coupling reagent.

7. Method according to claim 6, **characterized in that** the cyclisation reaction is carried out with HCTU or TCTU or with a phosphonium salt of the benzotriazol as the coupling agent.

8. Method according to claim 7, **characterized in that** the peptide is linked to a resin support by means of an acid-labile bond.

9. Method according to one of the claims 1 to 6, **characterized in that** the peptide is C-terminally covalently connected via an acid-labile bond.

10. Method according to claim 1, **characterized in that** the base-labile protection group is an Fmoc group.

11. Method according to claim 1 or 8, **characterized in that** the allyl and alloc protection group are removed by Pd(0) catalysis in the presence of an allyl acceptor.

12. Method according to claim 11, **characterized in that** an excess of (R1-Ph)ₙSiH₄₋ₙ or of PhSiH₃ is used as allyl acceptor, wherein R1 is a substitutent at the aromatic core and is aryl, alkyl or aralkyl, and n is 1 or 2.

13. A cyclic peptide of formula II or III having an Nα that is protected with a base-labile protection group, 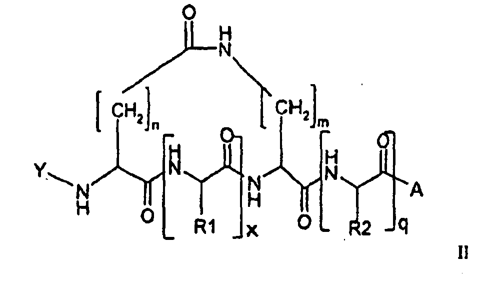 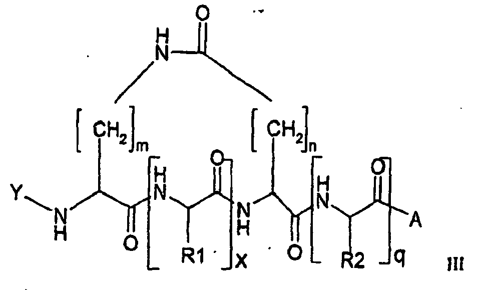 wherein Y is the base-labile protection group, n = 1-10, m = 1-15, x = 1-200 and q = 0-200, R1 and R2 each are, independently a natural amino acid side chain or non-natural derivative thereof, which side chain further may comprise a protection group with the exception of allyl ether and allyloxycarbonyl protection groups, and wherein A is a resin or resin handles or wherein R1 is defined above and R2 is a natural amino acid side chain or non-natural derivative thereof which side chain is bonded to a resin or resin handle via an ether, thioether, ester, thioester, amido or secondary or tertiary amino moiety and A is selected from the group consisting of OH, NH₂, NR1'H and NR1'R2', with R1' and R2' being independently C₁-C₄ alkyl.

14. Peptide according to claim 13, wherein n = 1 or 2.

15. Peptide according to claims 13 or 14, **characterized in that** the base-labile group is Fmoc.

16. Peptide according to one of claims 13 to 15, **characterized in that** A comprises a resin handle or resin linkage moiety with the exception of resin handles comprising an allyloxycarbonyl moiety.

17. Peptide according to claim 16, **characterized in that** the resin or resin handle is of formula IV 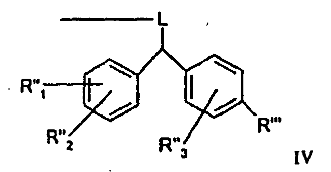 wherein R"' is a resin, and R1", R2", R3" are, independently, hydrogen, C₁-C₄ alkyl or C₁-C₄ alkoxy, and may be the same or different with the proviso that only one of R"1, R"2 may be hydrogen, and wherein L is oxygen, sulfur, nitrogen or is of formula V

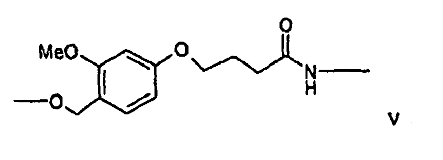

18. Peptide according to claim 17, wherein R"', R1" and R2" are as defined above, R3" is hydrogen and L is oxygen or nitrogen.

19. Peptide according to claim 18, **characterized in that** the resin or resin handle is of formula VII, 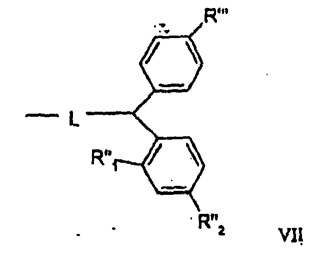 wherein R"', R1", R2" and L are as defined in claim 18.

20. Peptide according to claim 19, **characterized in that**, if L is oxygen, R1" and R2" are independently hydrogen, methyl or methoxy with the proviso that only one of R1", R2" may be hydrogen and, if L is nitrogen, R1" and R2" independently are methyl or methoxy.

21. Peptide according to claim 20, **characterized in that** L is oxygen, R1" is hydrogen and R2" is methyl or methoxy.

22. Peptide according to claim 21, wherein A is a resin or resin handle.

23. A cyclic peptide of formula 11 or III having an Nα that is protected with a base-labile protection group, 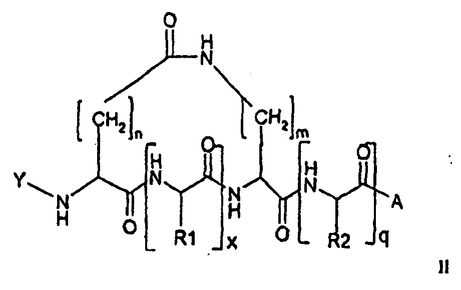 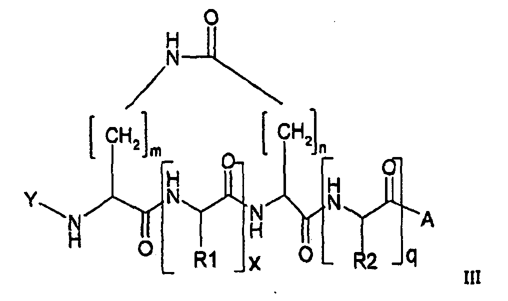 wherein Y is a base-labile protection group, n = 1-10, m = 1-15, x = 1-200 and q = 0-200, R1 and R2 each are, independently, a natural amino acid side chain or non-natural derivative thereof, which side chain further may comprise a protecting group with the exception of allyl ether and allyloxycarbonyl protecting groups, and wherein A is a resin or resin handle, or wherein R1 is as defined above and R2 is a natural amino acid side chain or non-natural derivative thereof which side chain is bonded to a resin or resin handle via an ether, thioether, ester, thioester, amido or, secondary or tertiary amino moiety and A is OR3' with R3' being a protection group with the exception of allyl groups.

24. Peptide according to claim 23, wherein R3' is tert-butyl or pentafluorophenyl.

25. Use of Pd(P[o-tolyl]₃)₂ or Pd(P[2,4-xylyl]₃)₂ complexes for catalyzing deprotection of allyl-protected carboxyl groups or allyloxycarbonyl protected amino and/or hydroxy groups.

26. Use according to claim 25, **characterized in that** an organic sulfinate is used as an allyl group acceptor or scavenger reagent.

27. Use according to claim 25 or 26, **characterized in that** the allyl- or allyloxycarbonyl-protected groups are part of an optionally further protected peptide.

28. Use according to claim 27, **characterized in that** the optionally further protected peptide is Fmoc protected wherein the Fmoc group is attached to the Nα of the peptide.

## Patentansprüche

1. Verfahren zur Zyklisierung eines Peptids durch Lactambildung, enthaltend die Schritte
(a) Entschützen des Peptids von Allyl-Typ-Schutzgruppen mittels Pd(0) Katalyse in Anwesenheit eines Allyl-Akzeptors, wobei das Peptid an seinem Nα mit einer basenlabilen Schutzgruppe geschützt ist und ausserdem mindestens eine Allyloxycarbonyl-geschützte Aminofunktion einer Lysinseitenkette oder eines Analogons einer derartigen Lysinseitenkette enthält und ausserdem mindestens eine Allylester-geschützte ω-Carboxylgruppe einer Glutamyl- oder Aspartylseitenkette oder eines Analogons einer derartigen Seitenkette enthält, während die basenlabile Schutzgruppe am Nα beibehalten wird, wobei besagte allylische Schutzgruppen substituiert oder unsubstituiert sind,
(b) Zyklisieren des Peptids durch Lactambildung besagter ungeschützter Seitenketten in Anwesenheit eines schwachen Basenreagenzes, und
(c) Entschützen des Peptids von der basenlabilen Schutzgruppe an seinem Nα.

2. Verfahren nach Anspruch 1, **dadurch gekennzeichnet, dass** das mit einer basenlabilen Schutzgruppe geschützte Nα das Nα eines Aspartylrestes ist, das die besagte weitere Allyester-geschützte ω-Carboxylgruppe besitzt.

3. Verfahren nach Anspruch 1 oder 2, wobei der Aspartylrest Teil der Dipeptidsequenz Asp-His ist.

4. Verfahren nach Anspruch 1, 2 oder 3, **dadurch gekennzeichnet, dass** es als einen weiteren Schritt (d) eine weitere Verlängerung des Peptids durch kontinuierliche Peptidsynthese oder Peptidsegmentkupplung des entschützten Nα enthält.

5. Verfahren nach Anspruch 4, **dadurch gekennzeichnet, dass** das Peptid während der Zyklisierung an eine Festphase gebunden ist mit der Massgabe, dass die allylischen Schutzgruppen nicht als eine Harzverbindungsgruppe dienen, und dass der Peptidverlängerungsschritt (d) eine fortgesetzte Festphasensynthese ist.

6. Verfahren nach Anspruch 1, 2 oder 3, **dadurch gekennzeichnet, dass** der Zyklisierungsreaktionsschritt (b) in Anwesenheit eines schwachen Basenreagenzes in einem aprotischen polaren Lösungsmittel und in der weiteren Anwesenheit eines Kupplungsreagenzes durchgeführt wird.

7. Verfahren nach Anspruch 6, **dadurch gekennzeichnet, dass** die Zyklisierungsreaktion mit HCTU oder TCTU oder mit einem Phosphoniumsalz des Benzotriazols als Kupplungsreagenz durchgeführt wird.

8. Verfahren nach Anspruch 7, **dadurch gekennzeichnet, dass** das Peptid mittels einer säurelabilen Bindung an einen Harzträger gebunden ist.

9. Verfahren nach einem der Ansprüche 1 bis 6, **dadurch gekennzeichnet, dass** das Peptid C-terminal durch eine säurelabile Bindung kovalent gebunden ist.

10. Verfahren nach Anspruch 1, **dadurch gekennzeichnet, dass** die basenlabile Schutzgruppe eine Fmoc-Gruppe ist.

11. Verfahren nach Anspruch 1 oder 8, **dadurch gekennzeichnet, dass** die Allyl- oder Alloc-Schutzgruppe durch Pd(0) Katalyse in Anwesenheit eines Allyl-Akzeptors entfernt wird.

12. Verfahren nach Anspruch 11, **dadurch gekennzeichnet, dass** ein Überschuss von (R¹-Ph)*ₙ*SiH_{4-*n*} oder von PhSiH₃ als Allyl-Akzeptor verwendet wird, wobei R¹ ein Substituent am aromatischen Kern ist und Aryl, Alkyl oder Aralkyl ist, und *n* 1 oder 2 ist.

13. Zyklisches Peptid der Formel II oder III, das ein Nα hat, das mit einer basenlabilen Schutzgruppe geschützt ist, 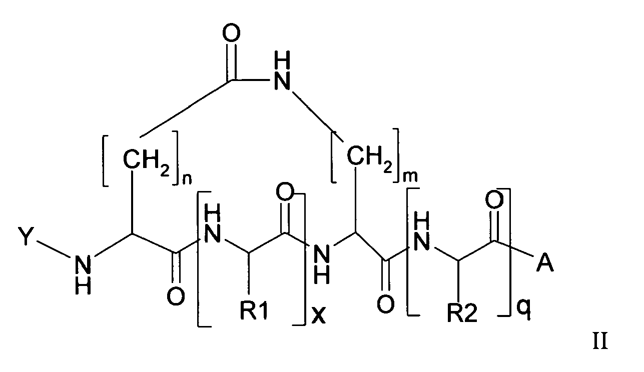 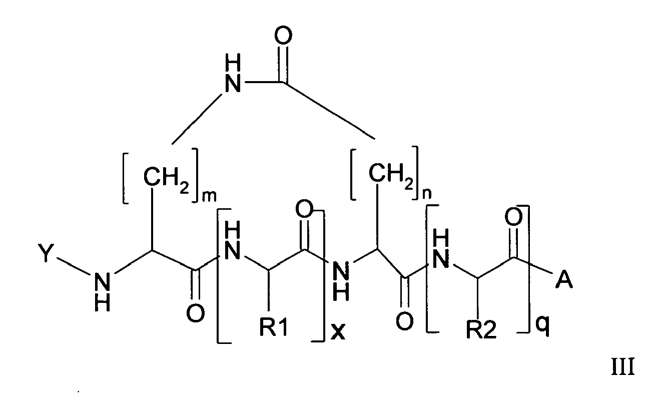 wobei Y die basenlabile Schutzgruppe ist, n = 1-10, m = 1-15, x = 1-200 und q = 0-200, R¹ und R² unabhängig voneinander eine natürliche Aminosäureseitenkette oder ein nicht-natürliches Derivat davon sind, wobei die Seitenkette ferner eine Schutzgruppe mit der Ausnahme einer Allylether- oder Allyloxycarbonyl-Schutzgruppe enthalten kann, und wobei A ein Harz oder eine Harzverbindungsgruppe ist, oder wobei R¹ wie oben definiert ist und R² eine natürliche Aminosäureseitenkette oder ein nicht-natürliches Derivat davon ist, die Seitenkette an das Harz oder an die Harzverbindungsgruppe durch eine Ether-, Thioether-, Ester-, Thioester-, Amido- oder sekundäre oder tertiäre Amino-Einheit gebunden ist und A aus der Gruppe bestehend aus OH, NH₂, NR^{1'}H und NR^{1'}R^{2'} ausgewählt ist, wobei R^{1'} und R^{2'} unabhängig voneinander C₁-C₄ Alkyl sind.

14. Peptid nach Anspruch 13, wobei n = 1 oder 2.

15. Peptid nach Anspruch 13 oder 14, **dadurch gekennzeichnet, dass** die basenlabile Gruppe Fmoc ist.

16. Peptid nach einem der Ansprüche 13 bis 15, **dadurch gekennzeichnet, dass** A eine Harzverbindungsgruppe oder Harzverbindungseinheit enthält, mit der Ausnahme von Harzverbindungsgruppen, die eine Allyloxycarbonyl-Einheit enthalten.

17. Peptid nach Anspruch 16, **dadurch gekennzeichnet, dass** das Harz oder die Harzverbindungsgruppe die Formel IV besitzt, 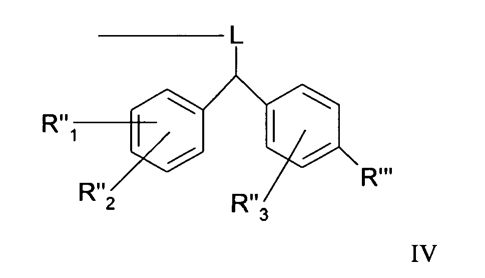 wobei R'" ein Harz ist, und R^{1"}, R^{2"}, R^{3"} unabhängig voneinander Wasserstoff, C₁-C₄ Alkyl oder C₁-C₄ Alkoxy sind, und gleich oder verschieden sein können, mit der Massgabe, dass nur eines von R^{1"}, R^{2"} Wasserstoff sein darf, und wobei L Sauerstoff, Schwefel oder Stickstoff ist oder die Formel V besitzt

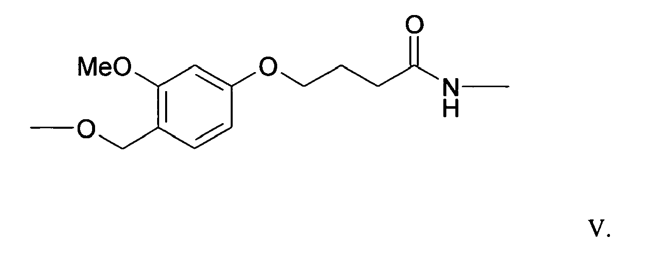

18. Peptid nach Anspruch 17, wobei R"', R^{1"} und R^{2"} wie oben definiert sind, R^{3"} Wasserstoff ist und L Sauerstoff oder Stickstoff ist.

19. Peptid nach Anspruch 18, **dadurch gekennzeichnet, dass** das Harz oder die Harzverbindungsgruppe die Formel VII besitzt, 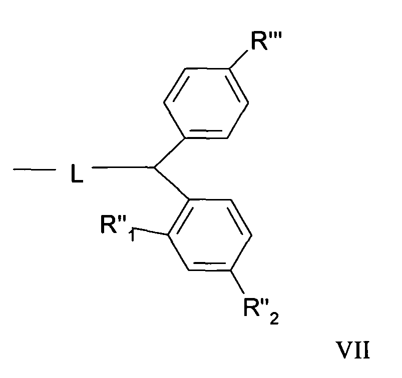 wobei R"', R^{1"}, R^{2"} und L wie in Anspruch 18 definiert sind.

20. Peptid nach Anspruch 19, **dadurch gekennzeichnet, dass**, falls L Sauerstoff ist, R^{1"} und R^{2"} unabhängig voneinander Wasserstoff, Methyl oder Methoxy sind, mit der Massgabe, dass nur eines von R^{1"}, R^{2"} Wasserstoff sein darf, und, falls L Stickstoff ist, R^{1"} und R^{2"} unabhängig voneinander Methyl oder Methoxy sind.

21. Peptid nach Anspruch 20, **dadurch gekennzeichnet, dass** L Sauerstoff ist, R^{1"} Wasserstoff ist und R^{2"} Methyl oder Methoxy ist.

22. Peptid nach Anspruch 21, wobei A ein Harz oder eine Harzverbindungsgruppe ist.

23. Zyklisches Peptid der Formel II oder III, das ein Nα hat, das mit einer basenlabilen Schutzgruppe geschützt ist, 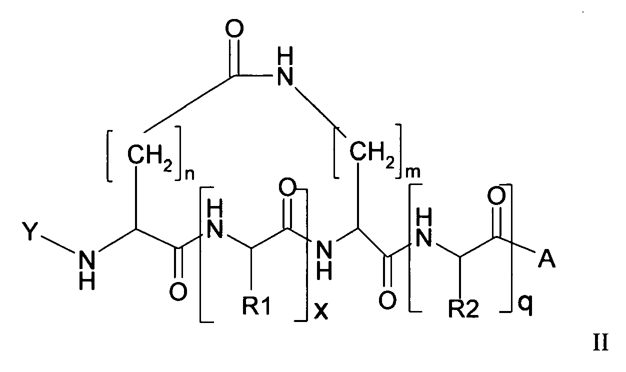 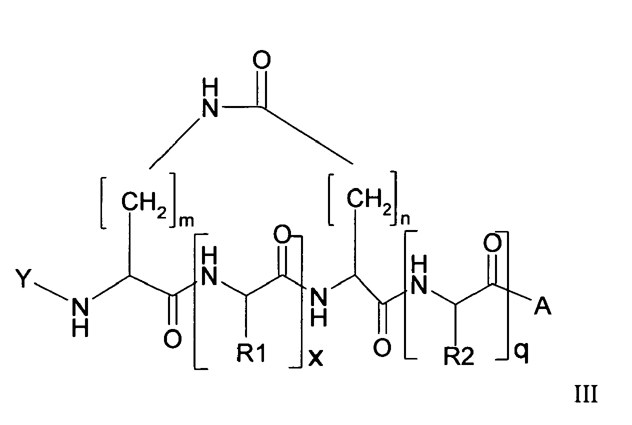 wobei Y die basenlabile Schutzgruppe ist, n = 1-10, m = 1-15, x = 1-200 und q = 0-200, R¹ und R² unabhängig voneinander eine natürliche Aminosäureseitenkette oder ein nicht-natürliches Derivat davon sind, wobei die Seitenkette ferner eine Schutzgruppe mit der Ausnahme einer Allylether- oder Allyloxycarbonyl-Schutzgruppe enthalten kann, und wobei A ein Harz oder eine Harzverbindungsgruppe ist, oder wobei R¹ wie oben definiert ist und R² eine natürliche Aminosäureseitenkette oder ein nicht-natürliches Derivat davon ist, die Seitenkette an das Harz oder an die Harzverbindungsgruppe durch eine Ether-, Thioether-, Ester-, Thioester-, Amido- oder sekundäre oder tertiäre Amino-Einheit gebunden ist und A OR^{3'} ist, wobei R^{3'} eine Schutzgruppe mit der Ausnahme von Allyl-Gruppen ist.

24. Peptid nach Anspruch 23, wobei R^{3'} *tert*-Butyl oder Pentafluorphenyl ist.

25. Verwendung von Pd(P[*o*-tolyl]₃)₂ oder Pd(P[2,4-xylyl]₃)₂ Komplexen zur Katalyse der Entschützung von Allyl-geschützten Carboxylgruppen oder Allyloxycarbonyl-geschützten Amino- und/oder Hydroxy-Gruppen.

26. Verwendung nach Anspruch 25, **dadurch gekennzeichnet, dass** ein organisches Sulfinat als ein Allylgruppen-Akzeptor- oder Abfängerreagenz verwendet wird.

27. Verwendung nach Anspruch 25 oder 26, **dadurch gekennzeichnet, dass** die Allyl- oder Allyloxycarbonyl-geschützten Gruppen Teil eines gegebenenfalls weiter geschützten Peptids sind.

28. Verwendung nach Anspruch 27, **dadurch gekennzeichnet, dass** das gegebenenfalls weiter geschützte Peptid Fmoc-geschützt ist, wobei die Fmoc-Gruppe an das Nα des Peptids geknüpft ist.

## Revendications

1. Procédé de cyclisation d'un peptide par lactamisation, comprenant les étapes consistant à
(a) déprotéger le peptide de groupes protecteurs de type allyle par catalyse par Pd(0) en présence d'un accepteur d'allyle, ledit peptide étant protégé avec un groupe protecteur labile en présence d'une base à son Nα et comprend en outre au moins une fonction amino protégée par un allyloxycarbonyle d'une chaîne latérale de lysine ou d'un analogue de ladite chaîne latérale de lysine et comprend en outre au moins un groupe ω-carboxyle protégé par un ester allylique d'une chaîne latérale de glutamyle ou d'aspartyle ou d'un analogue de ladite chaîne latérale tout en retenant le groupe protecteur labile en présence d'une base sur le Nα, où lesdits groupes protecteurs allyliques sont substitués ou non substitués,
(b) cycliser le peptide par lactamisation desdites chaînes latérales déprotégées en présence d'un réactif faiblement basique, et
(c) déprotéger le peptide du groupe protecteur labile en présence d'une base à son Nα.

2. Procédé selon la revendication 1, **caractérisé en ce que** le Nα protégé avec un groupe protecteur labile en présence d'une base est le Nα d'un résidu aspartyle ayant ledit groupe ω-carboxyle protégé par un ester allylique.

3. Procédé selon la revendication 1 ou 2, dans lequel le résidu aspartyle fait partie de la séquence dipeptidique Asp-His.

4. Procédé selon la revendication 1, 2 ou 3, **caractérisé en ce qu'**il comprend une étape supplémentaire consistant à (d) allonger plus avant le peptide au moyen d'une synthèse de peptide continue ou d'un couplage de segment peptidique du Nα déprotégé.

5. Procédé selon la revendication 4, **caractérisé en ce que** le peptide est lié à une phase solide durant la cyclisation, à condition que les groupes protecteurs allyliques ne servent pas d'attache de résine, et que l'allongement de peptide de l'étape (d) est une synthèse de peptide en phase solide continue.

6. Procédé selon la revendication 1, 2 ou 3, **caractérisé en ce que** l'étape de réaction de cyclisation b. est conduite en présence d'un réactif faiblement basique dans un solvant aprotique polaire et en outre en présence d'un réactif de couplage.

7. Procédé selon la revendication 6, **caractérisé en ce que** la réaction de cyclisation est conduite avec HCTU ou TCTU ou avec un sel de phosphonium du benzotriazole en tant qu'agent de couplage.

8. Procédé selon la revendication 7, **caractérisé en ce que** le peptide est lié à un support de résine au moyen d'une liaison labile en présence d'un acide.

9. Procédé selon l'une quelconque des revendications 1 à 6, **caractérisé en ce que** le peptide est lié de manière covalente au C-terminal par l'intermédiaire d'une liaison labile en présence d'un acide.

10. Procédé selon la revendication 1, **caractérisé en ce que** le groupe protecteur labile en présence d'un acide est un groupe Fmoc.

11. Procédé selon la revendication 1 ou 8, **caractérisé en ce que** les groupes protecteurs allyle et alloc sont enlevés par catalyse par Pd(0) en présence d'un accepteur d'allyle.

12. Procédé selon la revendication 11, **caractérisé en ce qu'**un excès de (R¹-Ph)*ₙ*SiH_{4-*n*} ou de PhSiH₃ est utilisé en tant qu'accepteur d'allyle, où R¹ est un substituant au niveau du noyau aromatique et est un aryle, un alkyle ou un aralkyle, et *n* est 1 ou 2.

13. Peptide cyclique de formule II ou III ayant un Nα qui est protégé avec un groupe protecteur labile en présence d'une base, 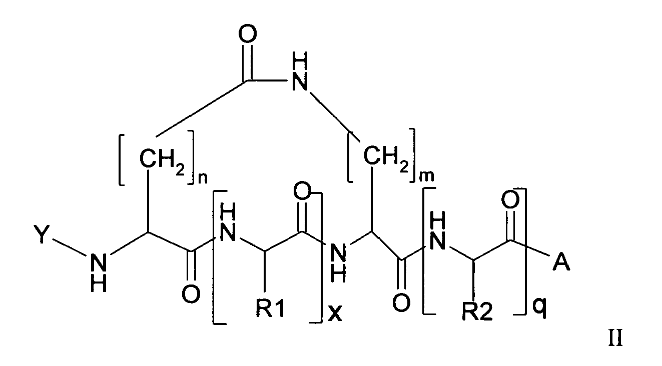 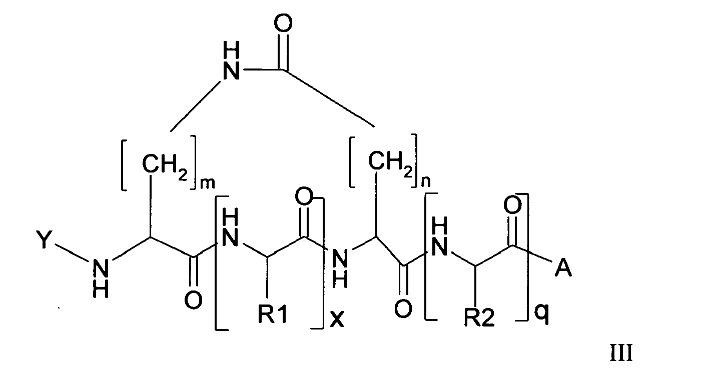 où Y est le groupe protecteur labile en présence d'une base, n = 1 à 10, m = 1 à 15, x = 1 à 200 et q = 0 à 200, R¹ et R² sont chacun indépendamment une chaîne latérale d'acide aminé naturel ou un dérivé non naturel de celle-ci, ladite chaîne latérale pouvant comprendre en outre un groupe protecteur à l'exception de groupes protecteurs d'éther allylique et allyloxycarbonyle, et où A est une résine ou une attache de résine ou bien où R¹ est comme défini ci-dessus et R² est une chaîne latérale d'acide aminé naturel ou un dérivé non naturel de celle-ci, ladite chaîne latérale étant liée à une résine ou attache de résine par l'intermédiaire d'un fragment éther, thioester, ester, thioester, amido ou amino secondaire ou tertiaire et A est choisi dans le groupe constitué de OH, NH₂, NR^{1'}H et NR^{1'}R^{2'}, R^{1'} et R^{2'} étant indépendamment un alkyle en C₁-C₄.

14. Peptide selon la revendication 13, dans lequel n = 1 ou 2.

15. Peptide selon les revendications 13 ou 14, **caractérisé en ce que** le groupe labile en présence d'une base est Fmoc.

16. Peptide selon une des revendications 13 à 15, **caractérisé en ce que** A comprend une attache de résine ou un fragment de liaison de résine à l'exception d'attaches de résine comprenant un fragment allyloxycarbonyle.

17. Peptide selon la revendication 16, **caractérisé en ce que** la résine ou attache de résine est de formule IV 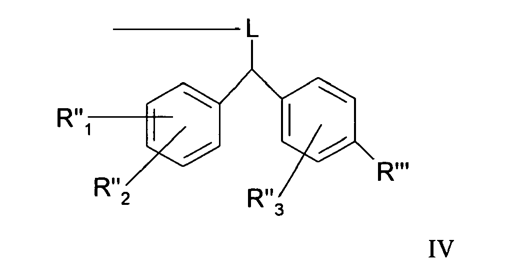 où R"' est une résine, et R^{1"}, R^{2"}, R^{3"} sont, indépendamment, un hydrogène, un alkyle en C₁-C₄ ou un alcoxy en C₁-C₄, et peuvent être identiques ou différents à condition qu'un seul parmi R^{1"} et R^{2"} peut être un hydrogène, et où L est un oxygène, un soufre, un azote ou est de formule V

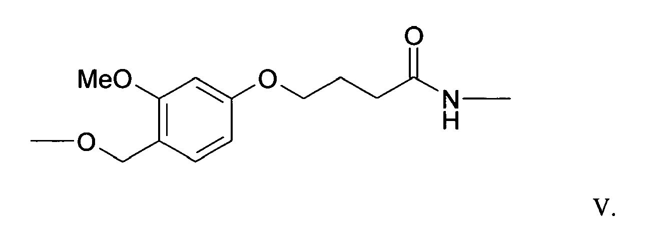

18. Peptide selon la revendication 17, où R"', R^{1"} et R^{2"} sont comme définis ci-dessus, R^{3"} est un hydrogène et L est un oxygène ou un azote.

19. Peptide selon la revendication 18, **caractérisé en ce que** la résine ou attache de résine est de formule VII, 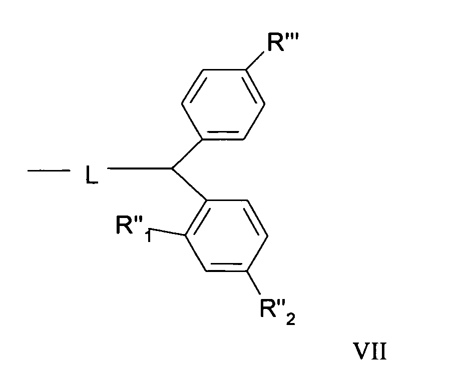 où R"', R^{1"}, R^{2"} et L sont comme définis dans la revendication 18.

20. Peptide selon la revendication 19, **caractérisé en ce que**, si L est un oxygène, R^{1"} et R^{2"} sont indépendamment un hydrogène, un méthyle ou un méthoxy à condition qu'un seul parmi R^{1"}, R^{2"} peut être un hydrogène et, si L est un azote, R^{1"} et R^{2"} sont indépendamment un méthyle ou un méthoxy.

21. Peptide selon la revendication 20, **caractérisé en ce que** L est un oxygène, R^{1"} est un hydrogène et R^{2"} est un méthyle ou un méthoxy.

22. Peptide selon la revendication 21, dans lequel A est une résine ou une attache de résine.

23. Peptide cyclique de formule II ou III ayant un Nα qui est protégé avec un groupe protecteur labile en présence d'une base, 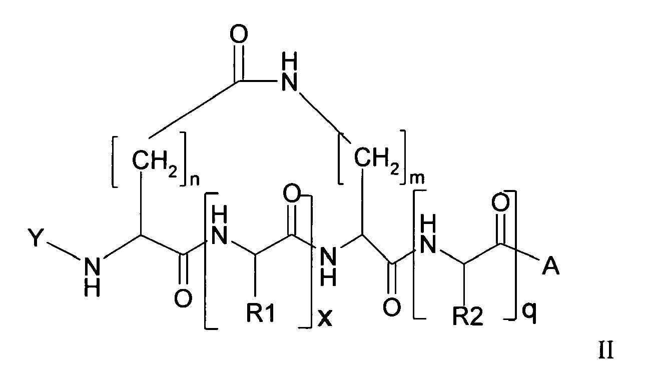 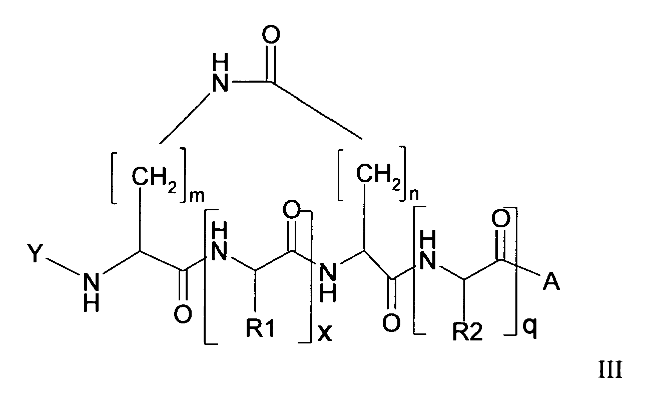 où Y est un groupe protecteur labile en présence d'une base, n = 1 à 10, m = 1 à 15, x = 1 à 200 et q = 0 à 200, R¹ et R² sont chacun indépendamment une chaîne latérale d'acide aminé naturel ou un dérivé non naturel de celle-ci, ladite chaîne latérale pouvant comprendre en outre un groupe protecteur à l'exception de groupes protecteurs d'éther allylique et allyloxycarbonyle, et où A est une résine ou attache de résine ou bien où R¹ est comme défini ci-dessus et R² est une chaîne latérale d'acide aminé naturel ou un dérivé non naturel de celle-ci, ladite chaîne latérale étant liée à une résine ou attache de résine par l'intermédiaire d'un fragment éther, thioester, ester, thioester, amido ou amino secondaire ou tertiaire et A est OR^{3'}, R^{3'} étant un groupe protecteur à l'exception de groupes allyle.

24. Peptide selon la revendication 23, dans lequel R^{3'} est un *tert*-butyle ou un pentafluorophényle.

25. Utilisation de complexes Pd(P[*o*-tolyle]₃)₂ ou Pd(P[2,4-xylyle]₃)₂ pour catalyser la déprotection de groupes carboxyle protégés par un allyle ou de groupes amino et/ou hydroxy protégés par un allyloxycarbonyle.

26. Utilisation selon la revendication 25, **caractérisée en ce qu'**un sulfinate organique est utilisé en tant que réactif fixateur ou accepteur de groupe allyle.

27. Utilisation selon la revendication 25 ou 26, **caractérisée en ce que** les groupes protégés par un allyle ou un allyloxycarbonyle font partie d'un peptide facultativement protégé plus avant.

28. Utilisation selon la revendication 27, **caractérisée en ce que** le peptide facultativement protégé plus avant est protégé par Fmoc, où le groupe Fmoc est lié au Nα du peptide.
